(19) Europäisches Patentamt
European Patent Office
Office européen des brevets

(11) EP 0 749 953 B1

(12) EUROPÄISCHE PATENTSCHRIFT

(45) Veröffentlichungstag und Bekanntmachung des Hinweises auf die Patenterteilung:
**17.11.1999 Patentblatt 1999/46**

(51) Int Cl.[6]: **C07C 53/19**, C07C 53/16, C07C 51/36

(21) Anmeldenummer: **96109401.8**

(22) Anmeldetag: **12.06.1996**

(54) **Herstellung von chiralen alpha-Halogencarbonsäuren**

Preparation of chiral alpha-halocarboxylic acids

Préparation d'acides alpha-halogénocarboxyliques chiraux

(84) Benannte Vertragsstaaten:
**AT BE CH DE DK ES FR GB IT LI NL**

(30) Priorität: **21.06.1995 CH 182095**

(43) Veröffentlichungstag der Anmeldung:
**27.12.1996 Patentblatt 1996/52**

(73) Patentinhaber: **Rolic AG**
**6301 Zug (CH)**

(72) Erfinder:
- **Broger, Emil Albin**
  **4312 Magden (CH)**
- **Buchecker, Richard**
  **8008 Zurich (CH)**
- **Crameri, Yvo**
  **4104 Oberwil (CH)**
- **Lukac, Teodor**
  **4054 Basel (CH)**

(74) Vertreter: **Scott, Fiona Penelope Elaine et al**
**BTG International Ltd**
**10 Fleet Place**
**Limeburner Lane**
**London EC4M 7SB (GB)**

(56) Entgegenhaltungen:
**EP-A- 0 272 787**
**WO-A-95/22405**
**US-A- 3 878 122**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 749 953 B1

## Beschreibung

Die Erfindung betrifft ein neues Verfahren zur Herstellung von optisch aktiven α-Brom- und α-Chlorcarbonsäuren bzw. deren Salzen oder Ester, durch enantioselektive Hydrierung von entsprechenden α, β-ungesättigten α-Brom- und α-Chlorcarbonsäure-Derivaten in Gegenwart eines optisch aktiven Rutheniumkomplexes.

Optisch aktive α-Brom- und α-Chlorcarbonsäuren sind wichtige und vielseitige Zwischenprodukte für die Synthese von pharmazeutischen Wirkstoffen oder für die Synthese von optisch aktiven Flüssigkristallen.

Enantioselektive Hydrierungen von α,β-ungesättigten α-Fluorcarbonsäuren mit Ruthenium-Binap-Komplexen sind bekannt und werden in Tetrahedron Letters 1992, 33, 7877 beschrieben.

Optisch aktive α-Brom- und α-Chlorcarbonsäuren wurden bisher aus optisch aktiven Aminosäuren oder α-Hydroxycarbonsäuren hergestellt. Es wurde nun gefunden, dass diese Verbindungen auf einfache Art und in hohen optischen Ausbeuten durch enantioselektive Hydrierung aus den entsprechenden Z-α,β-ungesättigten Edukten hergestellt werden können.

Bei einem in der EP-A-0 272 787 offenbarten Verfahren werden durch Hydrierung in Anwesenheit eines chiralen Katalysators Akrylsäurederivate, welche entweder in α-Stellung einfach substituiert und/oder in β-Stellung doppelt substituiert sind, in optisch aktive Propionsäurederivate überführt. Dabei sind die Substituenten in α-und/oder β-Stellung von ihrer Natur her keine Halogene sondern Kohlenwasserstoffreste.

Die Erfindung betrifft somit ein Verfahren zur Herstellung von optisch aktiven α-Brom- und α-Chlorcarbonsäuren bzw. deren Salzen oder Ester der allgemeinen Formel

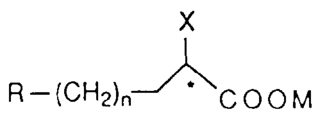

I

worin

| | |
|---|---|
| X | Brom oder Chlor; |
| M | Wasserstoff, $NR^{1'}_4{}^+$ oder ein Kation eines Alkali- oder Erdalkalimetalls; |
| $R^1$ | Wasserstoff oder niederes Alkyl; |
| n | 0 oder 1; |
| R | Wasserstoff, Alkyl mit 1 bis 20 Kohlenstoffatomen, welches gegebenenfalls endständig mit $-NR^2_2$, $-COOR^2$, $-OR^3$, einer freien oder geschützten -CHO-Gruppe oder einem Ring A substituiert sein kann; |
| $R^2$ | Wasserstoff oder niederes Alkyl; |
| $R^3$ | Wasserstoff oder eine Schutzgruppe; und |
| Ring A | einen gegebenenfalls substituierten Ring; |
| * | ein Chiralitätszentrum bedeuten, |

dadurch gekennzeichnet, dass ein α,β-ungesättigte Verbindung der allgemeinen Formel

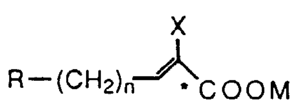

II

worin R, n, X und M wie oben definiert sind,

in Gegenwart eines Ruthenium-Komplexes eines optisch aktiven, vorzugsweise atropisomeren Diphosphinliganden enantioselektiv hydriert wird.

Im Rahmen der vorliegenden Erfindung bedeutet der Ausdruck "niederes Alkyl" einen geradkettigen oder verzweigten Alkyl-Rest mit 1 bis 5 Kohlenstoffatomen, wie beispielsweise Methyl, Ethyl, Propyl, i-Propyl, Butyl, i-Butyl, tert.-Butyl, Pentyl, i-Pentyl, und dergleichen.

Der Ausdruck "Alkyl mit 1 bis 20 Kohlenstoffatomen" umfasst im Rahmen der vorliegenden Erfindung geradkettige oder verzweigte Alkylreste mit 1 bis 20 Kohlenstoffatomen wie beispielsweise Methyl, Ethyl, Propyl, Butyl, Pentyl, Hexyl, Heptyl, Octyl, Nonyl, Decyl, Isopropyl, 2-Methyl-butyl, 2-Methyl-pentyl, 2-Methyl-hexyl, 2-Methyl-heptyl, 3-Methyl-butyl, 3-Methyl-pentyl, 3-Methyl-hexyl, 3-Methyl-heptyl, 4-Methyl-pentyl, 4-Methyl-hexyl, 5-Methyl-hexyl, und

dergleichen.

Als Schutzgruppen $R^3$ kommen die üblichen, leicht abspaltbaren etherbildenden Schutzgruppen für Alkohole, wie beispielsweise Benzyl, Allyl, Benzyloxymethyl, niederes Alkoxymethyl oder auch 2-Methoxylethoxymethyl und dergleichen, in Frage.

Der Ausdruck "geschützte -CHO Gruppe" umfasst beispielsweise Acetale, wie Diethylacetale, 1,3-Dioxane, 1,3-Dioxolane und dergleichen.

Der Ausdruck "gegebenenfalls substituierter Ring" umfasst im Zusammenhang mit Ring A gesättigte 5- oder 6-gliedrige Ringe, welche einfach substituiert sein können, wie beispielsweise Cyclopentyl oder Cyclohexyl; oder ungesättigte bzw. aromatische 5- oder 6-gliedrige Ringe, welche einfach substituiert sein können, wie beispielsweise Cyclopenten, Cyclohexen, Cyclohexadien, Phenyl, Furan, Thiophen, Pyridin, Pyrimidin und dergleichen; oder kondensierte Ringe, wie Naphthalin, Tetrahydronaphthalin, Indol, Benzimidazol, Chinolin, Benzopyran und dergleichen. Als Substituenten für die obengenannten Ringe kommen beispielsweise Niederalkyl, Niederalkoxy, welche gegebenenfalls enständig Hydroxy, $NH_2$-, Brom oder Iod tragen können, $-NR^2_2$, $-COOR^2$, $-OR^3$, eine freie oder geschützte -CHO-Gruppe, und an aromatischen Ringen auch Halogen, in Frage.

Als Ruthenium-Komplexe von atropisomeren Diphosphinen kommen, im Rahmen der vorliegenden Erfindung, insbesondere Verbindungen der allgemeinen Formeln in Frage

$$[RuL]^{2+}(Z)_2, \quad III \qquad Ru(Z^1)_2L \quad IV \qquad \text{oder} \qquad [Ru(Z^2)_{2-m}(L)(X)](Z^3)_m \quad V$$

worin

- Z $BF_4^{\ominus}$, $ClO_4^{\ominus}$, $B(Phenyl)_4^{\ominus}$ oder $PF_6^{\ominus}$;
- $Z^1$ Halogen oder die Gruppe $Y\text{-}COO^{\ominus}$ oder $Y\text{-}SO_3^{\ominus}$;
- Y niederes Alkyl, Phenyl, halogeniertes niederes Alkyl oder halogeniertes Phenyl;
- $Z^2$ Halogen;
- X Benzol, Hexamethylbenzol oder p-Cymol;
- m die Zahl 1 oder 2;
- $Z^3$ Halogen, $BF_4^{\ominus}$, $ClO_4^{\ominus}$ oder $B(Phenyl)_4^{\ominus}$; und
- L einen optisch aktiven, atropisomeren Diphosphin-Liganden bedeuten.

Bevorzugte optisch aktive atropisomere Diphosphin-Liganden L sind Verbindungen der allgemeinen Formeln

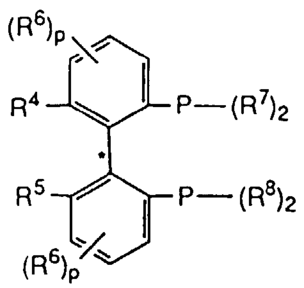

VI

und

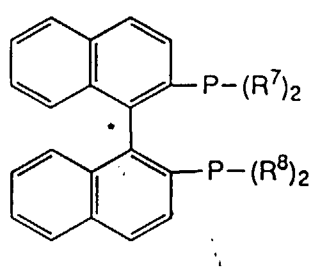

VII

worin

| | |
|---|---|
| $R^4$ und $R^5$ | unabhängig voneinander niederes Alkyl, niederes Alkoxy, Hydroxy, geschütztes Hydroxy oder zusammen $-O-CH_2-$, $-O-CH_2-O-$, |
| $R^6$ | niederes Alkyl oder niederes Alkoxy; |
| p | die Zahl 0, 1 oder 2; und |
| $R^7$ und $R^8$ | unabhängig voneinander niederes Alkyl, Cycloalkyl, Aryl, einen fünfgliedrigen Heteroaromaten oder zusammen mit dem Phosphoratom eine Gruppe der Formel |

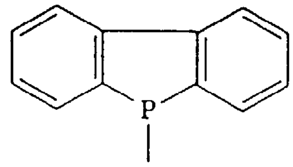

| | |
|---|---|
| | bedeuten; und |
| * | eine Chiralitätsachse bezeichnet. |

Die Verbindungen der Formeln VI und VII liegen in der (R)- oder (S)-Form vor.

Ebenfalls als optisch aktive Diphosphinliganden eignen sich für das erfindungsgemässe Verfahren Verbindungen der allgemeinen Formel VIII

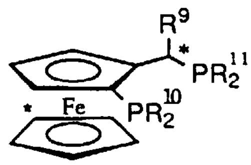

VIII

worin

| | |
|---|---|
| $R^9$ | Cycloalkyl oder niederes Alkyl; |
| $R^{10}$ | Aryl oder Heteroaryl; und |
| $R^{11}$ | Aryl, Heteroaryl oder niederes Alkyl bedeuten; und |
| * | ein Chiralitätszentrum bezeichnet. |

Der im Zusammenhang mit den Formeln III - VIII verwendete Ausdruck "niederes Alkyl" besitzt die oben definierte Bedeutung. Der Ausdruck "niederes Alkoxy" bedeutet Gruppen, in denen der Alkylrest die vorhergehende Bedeutung hat.

Der Ausdruck "halogeniertes niederes Alkyl" bedeutet im Rahmen der vorliegenden Erfindung niedere Alkylgruppen mit einer variablen Anzahl von Halogenatomen, insbesondere Chlor oder Fluor, wobei sich vorzugsweise wenigstens ein Halogenatom in α-Stellung zur -COO-Gruppe befindet. Bevorzugte halogenierte niedere Alkylgruppen sind perfluorierte und perchlorierte niedere Alkylgruppen, beispielsweise Trifluormethyl, Pentafluorethyl und dergleichen.

Der Ausdruck "halogeniertes Phenyl" umfasst, einfach oder mehrfach mit Fluor, Chlor, Brom oder Iod substituiertes Phenyl.

Der Ausdruck "Halogen" bedeutet im Rahmen der vorliegenden Erfindung Fluor, Chlor, Brom oder Jod.

Als Schutzgruppen für die Hydroxygruppen werden im Zusammenhang mit Verbindungen der Formeln III-VIII, insbesondere die üblichen etherbildenden Gruppen, wie z.B. Benzyl, Allyl, Benzyloxymethyl, niederes Alkoxymethyl oder auch 2-Methoxylethoxymethyl und dergleichen verwendet.

Der Ausdruck "Cycloalkyl" bezeichnet Ringe mit 3 bis 8 Kohlenstoffatomen, wie Cyclopropyl, Cyclobutyl, Cyclopentyl, Cyclohexyl oder Cycloheptyl.

Der Ausdruck "Aryl" bedeutet im Rahmen der vorliegenden Erfindung insbesondere den Phenylrest, welcher sowohl unsubstituiert als auch in ortho-, meta- oder para-Stellung einfach oder auch mehrfach substituiert sein kann. Geeignete Substituenten sind Phenyl, niedere Alkyl- oder niedere Alkoxygruppen, vorzugsweise Methyl- oder Methoxygruppen, Di-niederes Alkylamino, vorzugsweise Dimethylamino, Fluor, Trialkylsilyl, wie Trimethylsilyl, Sulfamoyl, wie N,N-Dimethylaminosulfamoyl, Sulfonyl oder ein Sulfonylsalz und dergleichen. Der Ausdruck kann zudem auch Naphthyl bedeuten. Der Ausdruck "halogeniertes Phenyl" bedeutet vorzugsweise Perfluorphenyl oder Perfluorbiphenyl.

Der Ausdruck "fünfgliedriger Heteroaromat" steht, im Rahmen der vorliegenden Erfindung, für einen Substituenten der allgemeinen Formel

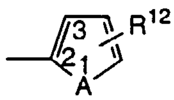 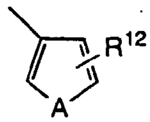 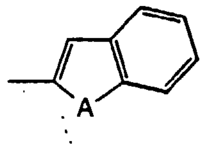 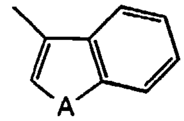

(a) (b) (c) oder (d)

In den Substituenten der Formeln (a) bis (d) bedeutet A Sauerstoff, Schwefel oder -$NR^{13}_2$. Der Substituent $R^{12}$ bedeutet Wasserstoff, niederes Alkyl, insbesondere Methyl, oder niederes Alkoxy, insbesondere Methoxy, und $R^{13}$ steht für niederes Alkyl, vorzugsweise Methyl.

Das Zeichen (*) bedeutet, dass es sich bei dem in Frage stehenden C-Atom um ein asymmetrisches C-Atom handelt.

Die erfindungsgemäss verwendeten Diphosphin-Liganden der Formeln VI, VII und VIII sind bekannte oder Analoge bekannter Verbindungen.

Die Komplexe der Formel III sind bekannt oder Analoge bekannter Verbindungen und können in an sich bekannter Weise, beispielsweise gemäss Takaya et al., J. Org. Chem. 1987, 52, 3174-3177, hergestellt werden.

Die Komplexe der Formeln IV und V sind ebenfalls bekannt oder Analoge bekannter Verbindungen, und können beispielsweise wie in EP 397 042 beschrieben hergestellt werden.

Die erfindungsgemässen asymmetrischen Hydrierungen erfolgen zweckmässig in einem, unter den Reaktionsbedingungen inerten, organischen Lösungsmittel oder in Wasser. So eignen sich beispielsweise niedere Alkohole, wie Methanol, Ethanol, Propanol und dergleichen; halogenierte Kohlenwasserstoffe, wie Dichlormethan, Chloroform, Hexafluorbenzol und dergleichen; Ether, wie Tetrahydrofuran, Dioxan und dergleichen; Ketone, wie Aceton, Diethylketon, Methylethylketon; Säuren, wie Essigsäure; Ester, wie Ethylacetat; Kohlenwasserstoffe, wie Pentan, Hexan und dergleichen; oder Gemische der obenerwähnten Lösungsmittel untereinander oder mit Wasser.

Das Verhältnis von Ruthenium zu Ligand L in den Komplexen der Formeln III, IV und V liegt zweckmässig zwischen etwa 0,5 und etwa 2 Mol, vorzugsweise bei etwa 1 Mol, Ruthenium pro Mol Ligand. Das Substrat/Katalysator-Verhältnis (S/C; Mol/Mol) liegt zweckmässig zwischen etwa 20 und etwa 30000, vorzugsweise zwischen etwa 100 und etwa 5000.

Die asymmetrische Hydrierung mit den Komplexen der Formeln III, IV und V erfolgt zweckmässig bei einer Temperatur von etwa 10° C bis etwa 100°C, vorzugsweise bei etwa 20°C bis etwa 60°C. Diese Hydrierung erfolgt zweckmässig unter Druck, vorzugsweise bei einem Druck von etwa $0{,}1\cdot10^6$ Pa (1 bar) bis etwa $10\cdot10^6$ Pa (100 bar), besonders bevorzugt von etwa $0{,}2\cdot10^6$ Pa (2 bar) bis etwa $8\cdot10^6$ Pa (80 bar).

Das erfindungsgemässe Verfahren eignet sich in hervorragender Weise zur Herstellung von Verbindungen der Formel I, worin R Wasserstoff, geradkettige Alkylreste mit 1 bis 12 Kohlenstoffatomen oder geradkettige Alkylreste, welche endständig mit einer Hydroxygruppe oder einer Gruppe der Formeln $(CH_3O)_2CH$-; $(C_2H_5O)_2CH$- oder einem gegebenenfalls substituierten Ring der Formel

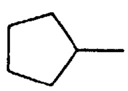 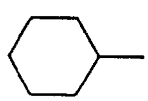 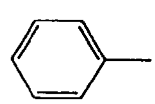 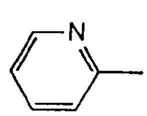 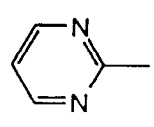

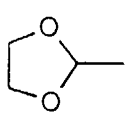 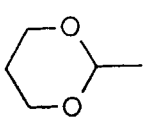 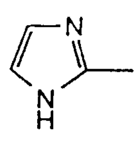 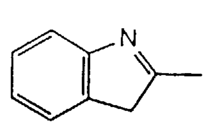 oder 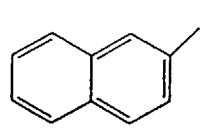

substituiert sein können. Als Substituenten der obengenannten Ringe kommen niedere Alkyl- oder niedere Alkoxyreste in Frage, welche gegebenenfalls endständig Hydroxy, $NH_2$-, Brom oder Iod tragen können; die 6-gliedrigen Ringe sind vorzugsweise in 4-Stellung, die 5-gliedrigen Ringe vorzugsweise in 3-Stellung und die kondensierten Ringe vorzugsweise in 6-Stellung substituiert.

Das erfindungsgemässe Verfahren ermöglicht auf einfache Weise den Zugang zu einer Reihe interessanter Zwischenprodukte, wie beispielsweise optisch aktiven α-Fluor- (1), α-Hydroxy- (2) und α-Aminocarbonsäuren bzw. ihrer Salze oder Ester (3), wie dies in Schema 1 dargestellt ist.

Schema 1

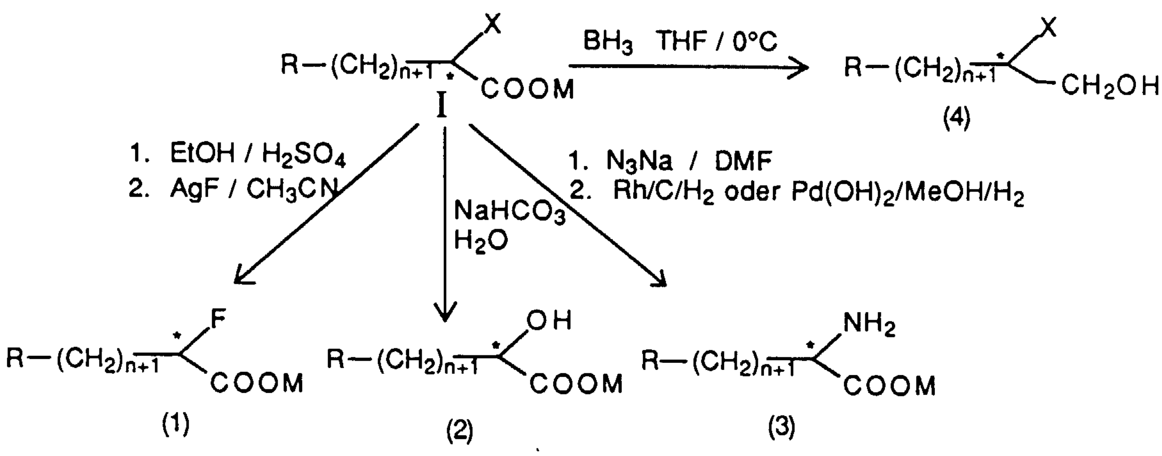


I (4) (1) (2) (3)

Die Umsetzung von Verbindungen der Formel I zu den entsprechenden α-Hydroxyderivaten (2) erfolgt direkt aus der freien Säure oder einem Salz. Für die Umsetzung von Verbindungen der Formel I zu den entsprechenden α-Fluor- (1) bzw. α-Aminocarbonsäuren (3) wird zweckmässig zunächst ein Ester gebildet, der anschliessend problemlos wieder gespalten werden kann. Die Verbindungen der Formel I können durch Reduktion, beispielsweise mit einem $BH_3$•THF-Komplex in die entsprechenden Alkohole (4) übergeführt werden.

Die als Edukt verwendeten Verbindungen der Formel II sind bekannt oder Analoge bekannter Verbindungen und sie können auf an sich bekannte Weise hergestellt werden.

Die folgenden Beispiele dienen zur Erläuterung der Erfindung und stellen keinerlei Einschränkungen dar. Im folgenden werden die verwendeten Abkürzungen erläutert:

| | |
|---|---|
| GC-FI % | Gaschromatographie, Flächenprozent |
| ee. | enantiomeren Ueberschuss |
| BIPHEMP | (6,6'-Dimethylbiphenyl-2,2'-diyl)bis(diphenylphosphin), |
| BINAP | [(1,1'-Binaphthyl)-2,2'-diyl]bis(diphenylphosphin), |
| $Cy_2$-MeOBIPHEP | (S)-P2,P2-Dicyclohexyl- 6,6'-dimethoxy-P2',P2'-diphenyl-biphenyl-2,2'-bis-phosphin, |
| 2-$Furyl_2$-BIPHEMP | P,P-Diphenyl-P',P'-di-2-furylphenyl)-(6,6'-dimethylbiphenyl-2,2'-diyl)diphosphin, |
| $Cy_4$-MeOBIPHEP | (6,6'-Dimethoxylbiphenyl-2,2'-diyl)bis(dicyclohexylphosphin), |
| (S)-(3,5-Ipr,4-MeO)-MeOBIPHEP $Ipr_4$-MeOBIPHEP | (S)-6,6'-Dimethyl-P,P,P',P'-tetrakis-(diisopropyl-4-methoxy-phenyl)-biphenyl-2,2'-bis phosphin, (6,6'-Dimethoxylbiphenyl-2,2'-diyl)bis(diisopropyllphosphin), |
| *(R)-(S)*-PPF-$P(tBu)_2$ | {*(R)*-1-[*(S)*-2-(Diphenylphosphino)ferrocenyl]}ethyl-di-tert.-butylphosphin, |
| MePHOS-MeOBIPHEP | [{2,5-Dimethyl-phospholan-1-yl}-6,6'-dimethoxybiphenyl-2-yl]-2,5-dimethyl-phospholan, |
| MeOBIPHEP-TS-Na | 6,6'-Dimethoxylbiphenyl-2,2'-bis(4,4'-phosphin-diyl-dibenzolsulfonsäure Natriumsalz. |

Beispiel 1

In einer Glove Box ($O_2$-Gehalt < 1ppm) wurden 7.4 mg (0.0226 mMol) Bis($\eta^2$-acetato)($\eta^4$-cycloocta-1,5-dien) ruthenium(II) und 12.5 mg (0.0226 mMol) *(R)*-BIPHEMP in einem Gemisch von 6 ml Diethylether und 2 ml Tetrahydrofuran gelöst und 1.5 Stunden bei 40° gerührt. In der Glove Box wurden dann 0.5 g (2.26 mMol) (*Z*)-2-Brom-2-octensäure

und 60 ml Methanol in einen 185 ml-Autoklav vorgelegt und die oben hergestellte Katalysatorlösung zugegeben. Der Autoklav wurde verschlossen und die Hydrierung unter Rühren bei 20° und einem konstanten Druck von $5 \cdot 10^6$ Pa (50 bar) durchgeführt. Die Hydrierung wurde nach 17 Stunden abgebrochen. Der Umsatz betrug 93%. Die Hydrierlösung wurde bei $50°/2 \cdot 10^3$ Pa 50°/20 mbar) eingedampft und der Rückstand im Kugelrohrofen bei 115°/20 Pa (115°/0.2 mbar) destilliert. Man erhielt 0.4 g (80%) eines Gemisches von 93% (*R*)-2-Bromoctansäure (94.3% ee) und 7% Edukt als farbloses Oel. Zur ee-Bestimmung wurde eine Probe des Produktes mit Diazomethan verestert und durch Gaschromatographie an einer chiralen Phase (permethyliertes -Cyclodextrin gemischt mit OV-61®, einem Produkt der Firma ,, Ohio Valley Speciality Chemical") analysiert.

Beispiel 2

In einer Glove Box ($O_2$-Gehalt < 1ppm) wurde eine Katalysatorlösung durch Lösen von 0.35 g (0.45 mMol) $Ru(OAc)_2$[(*R*)-BIPHEMP] in 20 ml Methanol hergestellt und 15 Min bei 20° gerührt. Dann wurden 10.0 g (45.2 mMol) (*Z*)-2-Brom-2-octensäure und 94 ml Methanol in einem 185 ml-Autoklav vorgelegt und die oben hergestellte Katalysatorlösung zugegeben. Der Autoklav wurde verschlossen und die Hydrierung unter Rühren bei 20° und einem konstanten Druck von $5 \cdot 10^6$ Pa (50 bar) durchgeführt. Nach 3 Stunden betrug der Umsatz 100%. Die Hydrierlösung wurde bei $50°/2 \cdot 10^3$ Pa (50°/20 mbar) eingedampft und der Rückstand im Kugelrohrofen bei 115°/20 Pa (115°/0.2 mbar) destilliert. Man erhielt 9.4 g (94%) eines Gemisches von 98.6% (*R*)-2-Brom-octansäure (93.8% ee) und 1.4% Edukt als farbloses Oel.

Beispiel 3

In einer Glove Box ($O_2$-Gehalt < 1ppm) wurde eine Katalysatorlösung durch Lösen von 59.6 mg (0.045 mMol) $Ru(CF_3COO)_2$[*(S)*-MeOBIPHEP-TS-Na] in 20 ml Wasser hergestellt und 15 Min. bei 20° gerührt. Dann wurden 1.0 g (4.52 mMol) *(Z)*-2-Brom-2-octensäure, 30 ml Wasser und 0.46 g (4.52 mMol) Triethylamin in einem 185 ml-Autoklav vorgelegt und die oben hergestellten 20 ml Katalysatorlösung zugegeben. Der Autoklav wurde verschlossen und die Hydrierung unter Rühren bei 20° und einem konstanten Druck von $5 \cdot 10^6$ Pa (50 bar) durchgeführt. Nach 4 Stunden betrug der Umsatz 100%. Die Hydrierlösung wurde mit 1N HCl-Lösung auf pH 1 angesäuert und mit Diethylether extrahiert. Nach Trocknung und Eindampfen ($40°/2 \cdot 10^3$ Pa bzw. 40°/20 mbar) des Etherextraktes wurde der Rückstand im Kugelrohrofen bei 115°/20 Pa (115°/0.2 mbar) destilliert. Man erhielt 0.9 g (90%) *(S)*-2-Brom-octansäure als farbloses Oel; ehem. Reinheit 98.9 GC-Fl%; 93.0% ee.

Beispiele 4 - 26

In zu Beispiel 1 oder 2 analoger Weise wurden die in Tabelle 1 aufgeführten Hydrierungen durchgeführt.

Tabelle 1:

| Asymmetrische Hydrierung von 2-Brom- und 2-Chlor-2-alkensäuren (R-CH=CXCOOM) | | | | | | | | | | |
|---|---|---|---|---|---|---|---|---|---|---|
| Bsp. | Konf. | X | R | M | T °C | Druck Pa x $10^6$ | Druck (bar) | Ligand L[1] | %Ums 20h | %ee |
| 4 | Z | Cl | n-$C_5H_{11}$ | H | 20 | 5 | (50) | *(R)*-BIPHEMP | 100 | 91.2 *(R)* |
| 5 | Z | Cl | n-$C_5H_{11}$ | H | 20 | 5 | (50) | *(S)*-BINAP | 97 | 76.3*(S)* |
| 6 | Z | Br | n-$C_4H_9$ | H | 20 | 5 | (50) | *(R)*-BIPHEMP | 100 | 94.1*(R)* |
| 7 | Z | Br | n-$C_6H_{13}$ | H | 20 | 5 | (50) | *(R)*-BIPHEMP | 100 | 94.4 *(R)* |
| 8 | Z | Br | n-$C_6H_{13}$ | H | 20 | 5 | (50) | *(R)*-$Cy_2$-MeOBIPHEP | 100 | 77.3 *(R)* |
| 9 | Z | Br | n-$C_6H_{13}$ | H | 20 | 5 | (50) | *(R)*-2-$Furyl_2$-BIPHEMP | 100 | 78.0 *(R)* |

[1] Katalysatorherstellung: analog Beispiel 2

Tabelle 1: (fortgesetzt)

| Asymmetrische Hydrierung von 2-Brom- und 2-Chlor-2-alkensäuren (R-CH=CXCOOM) | | | | | | | | | | |
|---|---|---|---|---|---|---|---|---|---|---|
| Bsp. | Konf. | X | R | M | T °C | Druck Pa x $10^6$ | Druck (bar) | Ligand L[1)] | %Ums 20h | %ee |
| 10 | Z | Br | n-$C_5H_{11}$ | H | 20 | 5 | (50) | *(S)*-(3,5-Ipr, 4-MeO)-MeOBIPHEP | 100 | 78.9 *(S)* |
| 11 | Z | Br | n-$C_5H_{11}$ | H | 20 | 5 | (50) | *(R,R,R)*-MePHOS-MeOBIPHEP [3)] | 24 | 78.6 *(R)* |
| 12 | Z | Br | n-$C_5H_{11}$ | H | 20 | 5 | (50) | *(R)*-$Ipr_4$-MeOBIPHEP | 69 | 84.8 *(R)* |
| 13 | Z | Br | n-$C_5H_{11}$ | H | 20 | 5 | (50) | *(R)*-$Cy_4$-MeOBIPHEP | 100 | 88.3 *(R)* |
| 14 | Z | Br | n-$C_5H_{11}$ | H | 20 | 5 | (50) | *(R)*-*(S)*-PPF-$P(tBu)_2$[2)] | 98 | 84.4 *(R)* |
| 15 | Z | Br | n-$C_3H_7$ | H | 20 | 5 | (50) | *(R)*-BIPHEMP | 99 | 92.1*(R)* |
| 16 | Z | Br | n-$C_7H_{15}$ | H | 20 | 5 | (50) | *(R)*-BIPHEMP | 100 | 93.6 *(R)* |
| 17 | Z | Br | n-$C_8H_{17}$ | H | 20 | 5 | (50) | *(S)*-BIPHEMP | 100 | 93.5 *(S)* |
| 18 | Z | Br | n-$C_5H_{11}$ | $NH_4^+$ | 20 | 5 | (50) | *(R)*-BIPHEMP | 100 | 90.7*(R)* |
| 19 | Z | Br | n-$C_5H_{11}$ | $Et_3NH^+$ | 20 | 5 | (50) | *(R)*-BIPHEMP | 100 | 87.3*(R)* |
| 20 | E | Cl | n-$C_5H_{11}$ | H | 20 | 5 | (50) | *(R)*-BIPHEMP | 100 | 10.1 *(R)* |
| 21 | E | Cl | n-$C_5H_{11}$ | H | 20 | 5 | (50) | *(S)*-BINAP | 100 | 9.0*(S)* |
| 22 | E | Br | n-$C_4H_9$ | H | 20 | 5 | (50) | *(R)*-BIPHEMP | 100 | 4.1*(S)* |
| 23 | Z | Br | n-$C_5H_{11}$ | H | 50 | 5 | (50) | *(R)*-BIPHEMP | 100 | 93.3*(R)* |
| 24 | Z | Br | n-$C_5H_{11}$ | H | 100 | 5 | (50) | *(S)*-BIPHEMP | 100 | 63.8*(S)* |
| 25 | Z | Br | n-$C_5H_{11}$ | H | 20 | 10 | (100) | *(S)*-BIPHEMP | 100 | 91.6*(S)* |
| 26 | Z | Br | n-$C_5H_{11}$ | H | 20 | 1 | (10) | *(S)*-BIPHEMP | 100 | 91.2 *(S)* |

[1)] Katalysatorherstellung: analog Beispiel 2
[2)] Katalysatorherstellung: in situ analog Beispiel 1
[3)] Herstellung nach M. Scalone, Referat am 9th International Symposium on Homogeneous Catalysis, Jerusalem, August 21-26, 1994.

Beispiel 27

In einer Glove Box ($O_2$-Gehalt < 1ppm) wurde eine Katalysatorlösung durch Lösen von 0.035 g (0.045 mMol) $Ru(OAc)_2$[*(R)*-BIPHEMP] und 17.2 mg (0.0905 mMol) einer 85proz. etherischen $HBF_4$-Lösung in 20 ml Methanol hergestellt und 1.5 Stunden bei 20° gerührt. Dann wurden 1.0 g (4.52 mMol) *(Z)*-2-Brom-2-octensäure und 105 ml Methanol in einem 185 ml-Autoklav vorgelegt und die oben hergestellte Katalysatorlösung zugegeben. Der Autoklav wurde verschlossen und die Hydrierung unter Rühren bei 50° und einem konstanten Druck von $5 \cdot 10^6$ Pa (50 bar) durchgeführt. Nach 21 Stunden betrug der Umsatz 98%. Die Hydrierlösung wurde bei $50°/2 \cdot 10^3$ Pa (50°/20 mbar) eingedampft und der Rückstand im Kugelrohrofen bei 115°/20 Pa (115°/0.2 mbar) destilliert. Man erhielt 0.6 g *(R)*-2-Brom-octansäure als farbloses Oel; ehem. Reinheit 97.3 GC-FI%; 86.0% ee.

<u>Beispiel 28</u>

In einer Glove Box ($O_2$-Gehalt < 1ppm) wurde eine Katalysatorlösung durch Lösen von 0.035 g (0.045 mMol) $Ru(OAc)_2$[*(R)*-BIPHEMP] und 1 ml einer 0.09 molaren methanolischen HCl-Lösung in 20 ml Methanol hergestellt und 1.5 Stunden bei 20° gerührt. Dann wurden 1.0 g (4.52 mMol) *(Z)*-2-Brom-2-octensäure und 105 ml Methanol in einem 185 ml-Autoklav vorgelegt und die oben hergestellte Katalysatorlösung zugegeben. Der Autoklav wurde verschlossen und die Hydrierung unter Rühren bei 50° und einem konstanten Druck von $5 \cdot 10^6$ Pa (50 bar) durchgeführt. Nach 21 Stunden betrug der Umsatz 87%. Die Hydrierlösung wurde bei $50°/2 \cdot 10^3$ Pa (50°/20 mbar) eingedampft und der Rückstand im Kugelrohrofen bei 115°/20 Pa (115°/0.2 mbar) destilliert. Man erhielt 0.6 g eines Gemisches von 86% *(R)*-2-Bromoctansäure (85.4% ee) und 14% Edukt als farbloses Oel.

## Patentansprüche

1. Verfahren zur Herstellung von optisch aktiven α-Brom- und α-Chlorcarbonsäuren bzw. deren Salzen oder Ester der Formel

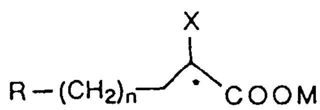

I

worin

| | |
|---|---|
| X | Brom oder Chlor; |
| M | Wasserstoff, $NR^1_4{}^+$ oder ein Kation eines Alkali- oder Erdalkalimetalls; |
| $R^1$ | Wasserstoff oder Alkyl mit 1 - 5 Kohlenstoffatomen; |
| n | 0 oder 1; |
| R | Wasserstoff, Alkyl mit 1 bis 20 Kohlenstoffatomen, welches gegebenenfalls endständig mit $-NR^2_2$, $-COOR^2$, $-OR^3$, einer freien oder geschützten -CHO-Gruppe oder einem Ring A substituiert sein kann; |
| $R^2$ | Wasserstoff oder Alkyl mit 1 - 5 Kohlenstoffatomen; |
| $R^3$ | Wasserstoff oder eine Schutzgruppe; |
| Ring A | ein gesättigter 5- oder 6-gliedriger Ring, welcher einfach substituiert sein kann, oder ein ungesättigter bzw. aromatischer 5-oder 6 gliedriger Ring, welcher einfach substituiert sein kann, oder ein kondensierter Ring; und |
| * | ein Chiralitätszentrum bedeuten, |

dadurch gekennzeichnet, dass eine α,β-ungesättigte Verbindung der Formel

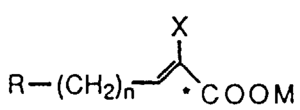

II

worin R, n, X und M wie oben definiert sind,
in Gegenwart eines Ruthenium-Komplexes eines optisch aktiven, vorzugsweise atropisomeren Diphosphinliganden enantioselektiv hydriert wird.

2. Verfahren gemäss Anspruch 1, dadurch gekennzeichnet, dass ein Rutheniumkomplex der Formel

$[RuL]^{2+}(Z)_2$, III $Ru(Z^1)_2L$ IV oder $[Ru(Z^2)_{2-m}(L)(X)](Z^3)_m$ V

verwendet wird, worin

Z $BF_4^{\ominus}$, $ClO_4^{\ominus}$, $B(Phenyl)_4^{\ominus}$ oder $PF_6^{\ominus}$;
$Z^1$ Halogen oder die Gruppe Y-$COO^{\ominus}$ oder Y-$SO_3^{\ominus}$;
Y Alkyl mit 1 - 5 Kohlenstoffatomen, Phenyl, halogeniertes Alkyl mit 1 - 5 Kohlenstoffatomen oder halogeniertes Phenyl;
$Z^2$ Halogen;
X Benzol, Hexamethylbenzol oder p-Cymol;
m die Zahl 1 oder 2;
$Z^3$ Halogen, $BF_4^{\ominus}$, $ClO_4^{\ominus}$ oder $B(Phenyl)_4^{\ominus}$; und
L einen optisch aktiven, atropisomeren Diphosphin-Liganden bedeuten.

**3.** Verfahren nach Anspruch 2, dadurch gekennzeichnet, dass der atropisomere Ligand L eine Verbindung der allgemeinen Formeln darstellt

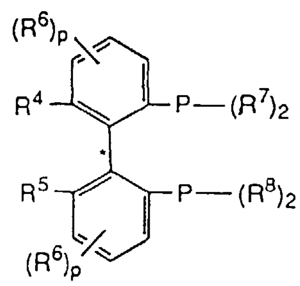

VI

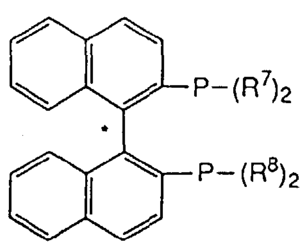

VII

und worin

$R^4$ und $R^5$ unabhängig voneinander Alkyl mit 1 - 5 Kohlenstoffatomen, Alkoxy mit einem Alkylrest enthaltend 1 - 5 Kohlenstoffatome, Hydroxy, geschütztes Hydroxy oder zusammen -O-$CH_2$-, -O-$CH_2$-O-,
$R^6$ Alkyl mit 1 - 5 Kohlenstoffatomen oder Alkoxy mit einem Alkylrest enthaltend 1 - 5 Kohlenstoffatome;
p die Zahl 0, 1 oder 2; und
$R^7$ und $R^8$ unabhängig voneinander Alkyl mit 1 - 5 Kohlenstoffatomen, einen Ring mit 3 - 8 Kohlenstoffatomen, Aryl, einen fünfgliedrigen Heteroaromaten oder zusammen mit dem Phosphoratom eine Gruppe der Formel

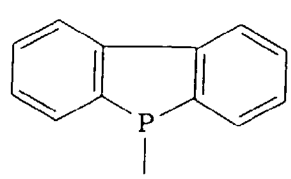

bedeuten; und
* eine Chiralitätsachse bezeichnet;

und welche in der (R)- oder (S)-Form vorliegen.

4. Verfahren nach Anspruch 2, dadurch gekennzeichnet, dass der atropisomere Ligand L eine Verbindung der allgemeinen Formel darstellt

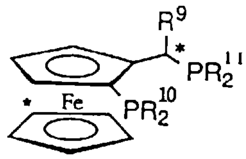

VIII

worin

$R^9$ einen Ring mit 3 bis 8 Kohlenstoffatomen oder Alkyl mit 1 - 5 Kohlenstoffatomen;
$R^{10}$ Aryl oder Heteroaryl; und
$R^{11}$ Aryl, Heteroaryl oder Alkyl mit 1 - 5 Kohlenstoffatomen bedeuten; und
* ein Chiralitätszentrum darstellt.

5. Verfahren gemäss einem der Ansprüche 1 bis 4, dadurch gekennzeichnet, dass das Verhältnis von Ruthenium zu Ligand L in den Komplexen der Formeln III, IV und V zwischen etwa 0,5 und etwa 2 Mol liegt.

6. Verfahren gemäss einem der Ansprüche 1 bis 4, dadurch gekennzeichnet, dass das Substrat/Katalysator-Verhältnis (S/C; Mol/Mol) zwischen etwa 20 und etwa 30000 liegt.

7. Verfahren gemäss einem der Ansprüche 1 bis 4, dadurch gekennzeichnet, dass die Reaktion bei einer Temperatur von etwa 20°C bis etwa 60°C und einem Druck von etwa $0{,}2 \cdot 10^6$ Pa bis etwa $8 \cdot 10^6$ Pa durchgeführt wird.

8. Verfahren gemäss einem der Ansprüche 1 bis 4, dadurch gekennzeichnet, dass eine Verbindung der Formel II, worin R Wasserstoff, geradkettige Alkylreste mit 1 bis 12 Kohlenstoffatomen oder geradkettige Alkylreste, welche endständig mit einer Hydroxygruppe oder einer Gruppe der Formeln $(CH_3O)_2CH$-, $(C_2H_5O)_2CH$- oder einem gegebenenfalls substituierten Ring der Formel

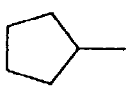 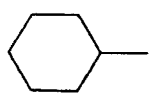 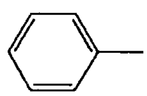 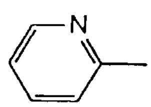 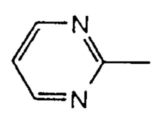

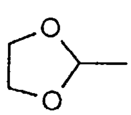 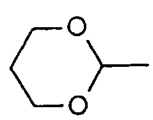 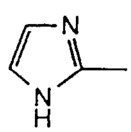 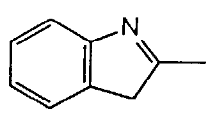 oder 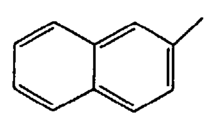

substituiert ist, hydriert wird.

## Claims

1. Process for the preparation of optically active α-bromo- and α-chloro-carboxylic acids and salts or esters thereof of formula

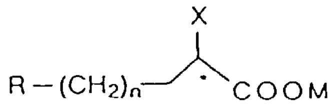

I

wherein

- X represents bromine or chlorine;
- M represents hydrogen, $NR^1_4{}^+$ or a cation of an alkali metal or of an alkaline earth metal;
- $R^1$ represents hydrogen or alkyl having from 1 to 5 carbon atoms;
- n represents 0 or 1;
- R represents hydrogen or alkyl having from 1 to 20 carbon atoms which may optionally be substituted in the terminal position by $-NR^2_2$, $-COOR^2$, $-OR^3$, a free or protected -CHO group or by a ring A;
- $R^2$ represents hydrogen or alkyl having from 1 to 5 carbon atoms;
- $R^3$ represents hydrogen or a protecting group;
- ring A is a saturated 5- or 6-membered ring which may be mono-substituted, or an unsaturated or aromatic 5- or 6-membered ring which may be mono-substituted, or a condensed ring; and
- * denotes a centre of chirality,

characterised in that an $\alpha,\beta$-unsaturated compound of formula

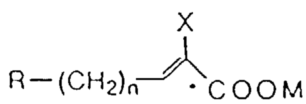

II

wherein R, n, X and M are as defined above,
is hydrogenated enantioselectively in the presence of a ruthenium complex of an optically active, preferably atropisomeric, diphosphine ligand.

**2.** Process according to claim 1, characterised in that a ruthenium complex of formula

$$[RuL]^{2+}(Z)_2, \quad III \qquad Ru(Z^1)_2L \quad IV \qquad or \qquad [Ru(Z^2)_{2-m}(L)(X)](Z^3)_m \quad V$$

is used, wherein

- Z represents $BF_4^{\ominus}$, $ClO_4^{\ominus}$, $B(phenyl)_4^{\ominus}$ or $PF_6^{\ominus}$;
- $Z^1$ represents halogen or the group $Y\text{-}COO^{\ominus}$ or $Y\text{-}SO_3^{\ominus}$;
- Y represents alkyl having from 1 to 5 carbon atoms, phenyl, halogenated alkyl having from 1 to 5 carbon atoms or halogenated phenyl;
- $Z^2$ represents halogen;
- X represents benzene, hexamethylbenzene or p-cymene;
- m represents the number 1 or 2;
- $Z^3$ represents halogen, $BF_4^{\ominus}$, $ClO_4^{\ominus}$ or $B(phenyl)_4^{\ominus}$; and
- L represents an optically active atropisomeric diphosphine ligand.

**3.** Process according to claim 2, characterised in that the atropisomeric ligand L is a compound of the general formula

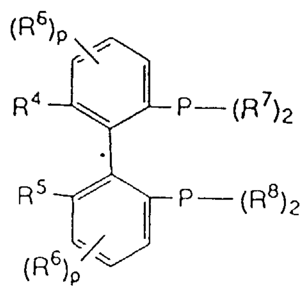

VI

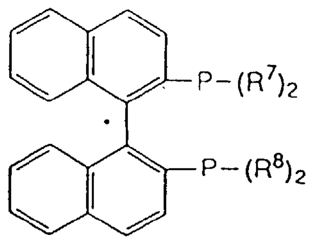

VII

and wherein

R⁴ and R⁵ each independently of the other represent alkyl having from 1 to 5 carbon atoms, alkoxy having an alkyl moiety containing from 1 to 5 carbon atoms, hydroxy, protected hydroxy, or together represent $-O-CH_2-$ or $-O-CH_2-O-$,

$R^6$ represents alkyl having from 1 to 5 carbon atoms or alkoxy having an alkyl moiety containing from 1 to 5 carbon atoms;

p represents the number 0, 1 or 2; and

$R^7$ and $R^8$ each independently of the other represent alkyl having from 1 to 5 carbon atoms, a ring having from 3 to 8 carbon atoms, aryl, a five-membered hetero aromatic, or together with the phosphorus atom represent a group of the formula

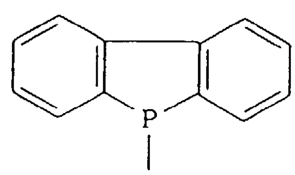

,

and

* denotes an axis of chirality;

and which is in the (R) or (S) form.

**4.** Process according to claim 2, characterised in that the atropisomeric ligand L is a compound of the general formula

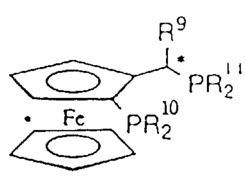

VIII

wherein

$R^9$ represents a ring having from 3 to 8 carbon atoms or alkyl having from 1 to 5 carbon atoms;
$R^{10}$ represents aryl or heteroaryl; and
$R^{11}$ represents aryl, heteroaryl or alkyl having from 1 to 5 carbon atoms; and
* denotes a centre of chirality.

**5.** Process according to any one of claims 1 to 4, characterised in that the ratio of ruthenium to ligand L in the complexes of formulae III, IV and V is from about 0.5 to about 2 mol.

**6.** Process according to any one of claims 1 to 4, characterised in that the substrate/catalyst ratio (S/C; mol/mol) is from about 20 to about 30 000.

**7.** Process according to any one of claims 1 to 4, characterised in that the reaction is carried out at a temperature of from about 20°C to about 60°C and at a pressure of from about $0.2 \times 10^6$ Pa to about $8 \times 10^6$ Pa.

**8.** Process according to any one of claims 1 to 4, characterised in that a compound of formula II wherein R represents hydrogen, straight-chain alkyl radicals having from 1 to 12 carbon atoms or straight-chain alkyl radicals that are substituted in the terminal position by a hydroxy group or a group of formula $(CH_3)_2CH$- or $(C_2H_5O)_2CH$- or by an optionally substituted ring of formula

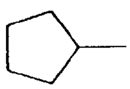 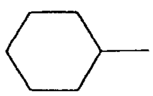 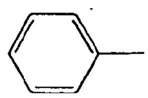 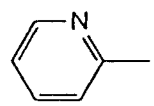 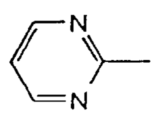

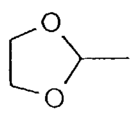 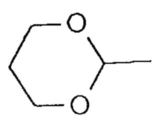 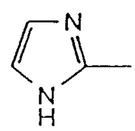 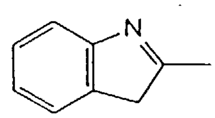 or 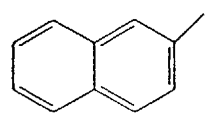

is hydrogenated.

## Revendications

**1.** Procédé pour la préparation d'acides α-bromo- et α-chloro-carboxyliques optiquement actifs et de leurs sels ou esters de formule

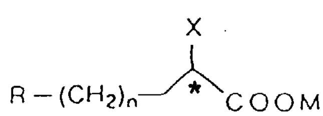 I

dans laquelle

| | |
|---|---|
| X | représente un atome de brome ou de chlore; |
| M | représente un atome d'hydrogène, $NR^1_4{}^+$ ou un cation d'un métal alcalin ou alcalino-terreux; |
| $R^1$ | représente un atome d'hydrogène ou un groupe alkyle ayant de 1 à 5 atomes de carbone; |
| n | représente 0 ou 1; |
| R | représente un atome d'hydrogène, un groupe alkyle ayant de 1 à 20 atomes de carbone, qui peut être éventuellement substitué en bout de chaîne par $-NR^2_2$, $-COOR^2$, $-OR^3$, un groupe -CHO libre ou protégé ou un cycle A; |
| $R^2$ | représente un atome d'hydrogène ou un groupe alkyle ayant de 1 à 5 atomes de carbone; |
| $R^3$ | représente un atome d'hydrogène ou un groupe protecteur; |
| le cycle A | représente un cycle saturé à 5 ou 6 chaînons, qui peut être monosubstitué, ou un cycle insaturé ou aromatique à 5 ou 6 chaînons, qui peut être monosubstitué, ou un cycle condensé; et |

* représente un centre chiral,

caractérisé en ce qu'on effectue une hydrogénation énantiosélective d'un composé $\alpha,\beta$-insaturé de formule

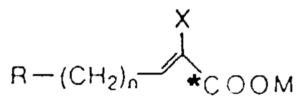

II

dans laquelle R, n, X et M sont comme définis ci-dessus, en présence d'un complexe du ruthénium avec un ligand diphosphine optiquement actif, de préférence atropo-isomère.

**2.** Procédé selon la revendication 1, caractérisé en ce qu'on utilise un complexe du ruthénium des formules :

$[RuL]^{2+}(Z)_2$, III $\quad Ru(Z^1)_2L$ IV ou $[Ru(Z^2)_{2-m}(L)(X)](Z^3)_m$ V

dans lesquelles

- Z représente $BF_4^{\ominus}$, $ClO_4^{\ominus}$, $B(phényle)_4^{\ominus}$ ou $PF_6^{\ominus}$;
- $Z^1$ représente un atome d'halogène ou le groupe $Y\text{-}COO^{\ominus}$ ou $Y\text{-}SO_3^{\ominus}$;
- Y représente un groupe alkyle ayant de 1 à 5 atomes de carbone, phényle, alkyle halogéné ayant de 1 à 5 atomes de carbone ou phényle halogéné;
- $Z^2$ représente un atome d'halogène;
- X représente un groupe benzène, hexaméthylbenzène ou p-cymène;
- m représente 1 ou 2;
- $Z^3$ représente un atome d'halogène, $BF_4^{\ominus}$, $ClO_4^{\ominus}$ ou $B(phényle)_4^{\ominus}$; et
- L représente un ligand diphosphine atropo-isomère, optiquement actif.

**3.** Procédé selon la revendication 2, caractérisé en ce que le ligand atropo-isomère L représente un composé des formules générales

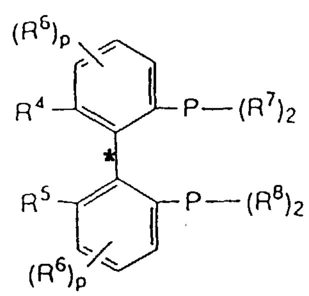

VI

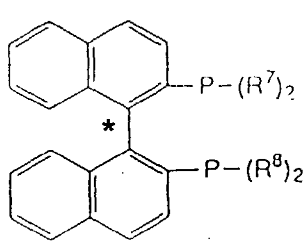

VII

et dans lesquelles

$R^4$ et $R^5$ représentent indépendamment l'un de l'autre un groupe alkyle ayant de 1 à 5 atomes de carbone, alcoxy avec un groupe alkyle ayant de 1 à 5 atomes de carbone, hydroxy, hydroxy protégé ou représentent ensemble $-O-CH_2-$, $-O-CH_2-O-$,

$R^6$ représente un groupe alkyle ayant de 1 à 5 atomes de carbone ou alcoxy avec un groupe alkyle ayant de 1 à 5 atomes de carbone;

p représente 0, 1 ou 2; et

$R^7$ et $R^8$ représentent indépendamment l'un de l'autre un groupe alkyle ayant de 1 à 5 atomes de carbone, un cycle ayant de 3 à 8 atomes de carbone, un groupe aryle, un composé hétéroaromatique à cinq chaînons ou représentent ensemble avec l'atome de phosphore un groupe de formule

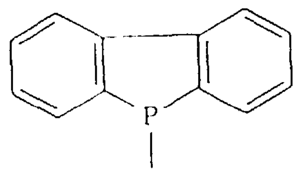

et

* désigne un axe de chiralité;

et qui se présentent sous la forme (R) ou (S).

**4.** Procédé selon la revendication 2, caractérisé en ce que le ligand L atropo-isomère représente un composé de formule générale

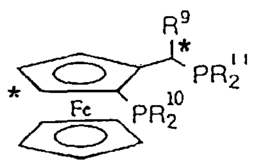

VIII

dans laquelle

$R^9$ représente un cycle ayant de 3 à 8 atomes de carbone ou un groupe alkyle ayant de 1 à 5 atomes de carbone;

$R^{10}$ représente un groupe aryle ou hétéroaryle; et

$R^{11}$ représente un groupe aryle, hétéroaryle ou alkyle ayant de 1 à 5 atomes de carbone; et

* représente un centre de chiralité.

**5.** Procédé selon l'une des revendications 1 à 4, caractérisé en ce que la proportion de ruthénium par rapport au ligand L dans les complexes de formules III, IV et V se situe entre environ 0,5 et environ 2 mol.

**6.** Procédé selon l'une des revendications 1 à 4, caractérisé en ce que le rapport substrat/catalyseur (S/C; mol/mol) se situe entre environ 20 et environ 30000.

**7.** Procédé selon l'une des revendications 1 à 4, caractérisé en ce qu'on réalise la réaction à une température d'environ 20°C à environ 60°C et sous une pression d'environ $0{,}2 \cdot 10^6$ Pa jusqu'à environ $8 \cdot 10^6$ Pa.

**8.** Procédé selon une des revendications 1 à 4, caractérisé en ce qu'on réalise l'hydrogénation d'un composé de formule II, dans laquelle R représente un atome d'hydrogène, un groupe alkyle linéaire ayant de 1 à 12 atomes de carbone ou un groupe alkyle linéaire, qui est substitué en bout de chaîne par un groupe hydroxy ou un groupe de formules $(CH_3O)_2CH-$, $(C_2H_5O)_2CH-$ ou par un cycle éventuellement substitué des formules

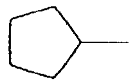 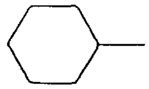 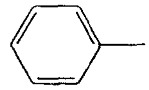 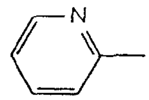 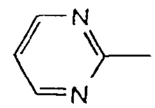

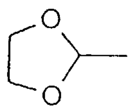 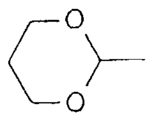 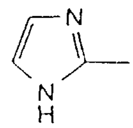 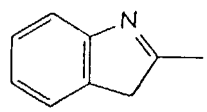 or 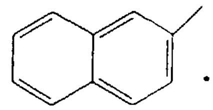 .